Europäisches Patentamt

European Patent Office

Office européen des brevets

(11) Publication number: **0 411 590 A2**

# EUROPEAN PATENT APPLICATION

(21) Application number: 90114720.7

(22) Date of filing: 31.07.90

(51) Int. Cl.⁵: **A61K 31/60**, A61K 9/24, A61K 9/54

(30) Priority: 02.08.89 US 388766

(43) Date of publication of application:
06.02.91 Bulletin 91/06

(84) Designated Contracting States:
AT BE CH DE DK ES FR GB GR IT LI LU NL SE

(71) Applicant: ARNOLD, John David
5801 Howe Drive
Fairway, Kansas 66205(US)

(72) Inventor: ARNOLD, John David
5801 Howe Drive
Fairway, Kansas 66205(US)

(74) Representative: Baillie, Iain Cameron et al
c/o Ladas & Parry Isartorplatz 5z 5
D-8000 München 2(DE)

(54) Method and preparation for reducing risk of myocardial infarction.

(57) A method of, and preparation for, treatment of human patients to inhibit platelet aggregation is provided. Generally, the method comprises the administration of acetylsalicylic acid (ASA) to reduce risk of myocardial infarction. The method comprises an administration once daily of a preparation permitting two pulses of ASA to be absorbed, the first pulse preferably being absorbed within two hours after administration and the second pulse being absorbed about six hours to eighteen hours after administration. In the preferred embodiment, the method comprises a total administration of 300 milligrams of ASA, once daily, with the first pulse preferably containing about one-third of the ASA. The method will inhibit platelet aggregation, while at the same time generally minimizing undesirable effects such as gastrointestinal bleeding. A preparation is provided for use with the method comprising a pill with a central core containing about 200 milligrams of ASA, surrounded by a protective coating preventing absorption of the central core until between about six hours and eighteen hours after administration. Adhering to the central core and protective coating is an outer layer of ASA prepared for absorption into the portal circulatory system during a time period of within about two hours following administration of the pill.

# METHOD AND PREPARATION FOR REDUCING RISK OF MYOCARDIAL INFARCTION

## Background of the Invention

The present invention relates to myocardial infarction (MI) prophylaxis and in particular to the use of acetylsalicylic acid (ASA) to inhibit certain biological processes which can lead to, or increase the risk of, myocardial infarction. Specifically, the invention involves a method of treatment, and a preparation for treatment, wherein acetylsalicylic acid (aspirin) is given to a patient. The methodology of the invention concerns a general avoidance of, or reduction of, gastrointestinal discomfort and bleeding, while at the same time providing a multiple pulse, or charge, of acetylsalicylic acid (ASA) into the biological system.

Cardiovacular disease is a major killer of humans. Myocardial infarction generally accounts for the single largest subgroup of cardiovascular deaths and, therefore, has been of particular concern. For example, it has been estimated that in 1984 over 1,500,000 persons suffered from myocardial infarction, with approximately 550,000 of them dying either from primary or subsequent MI.

There is considerable evidence that blood platelets play a key role in two well-recognized contributing processes to myocardial infarction: atherosclerosis; and thrombogenesis. Generally, atherosclerosis of the coronary arteries has been found in almost all patients who die of myocardial infarction. Further, platelets are integral components of coronary thrombi, which are important in the pathogenesis of acute myocardial infarction.

With respect to atherosclerosis, the deterioration process is apparently initiated by damage to the endothelial lining of the arteries. The endothelial lining forms a barrier that prevents passage of blood constituents into the arterial wall. Damage to the endothelium can occur in many ways, with the result often being that endothelial cells are dislodged from the arterial wall. This exposes the subendothelial connective tissue to platelets, which then can interact with collagen in the connective tissue.

The result of collagen interaction with platelets can be the release of a number of substances that stimulate platelet aggregation. In particular, apparently there is some stimulation of cyclooxygenase, which is located in the platelet membrane and which is involved in initiation of the arachidonic acid cycle leading to platelet aggregation. The result of platelet aggregation, of course, being the possible formation of a clot which can form a plug in narrowed arteries.

With respect to thrombosis: it is noted that acute necrotic changes take place in the myocardium following a catastrophic reduction in coronary blood supply. Generally, again, biological processes are initiated which generate platelet aggregation and form the clot, or thrombis. When the thrombis is detached from its original site, a thrombotic embolus may result, leading to myocardial infarction.

Generally, prophylactic treatment for myocardial infarction has centered around introduction of substances to inhibit platelet aggregation, i.e. inhibit clot formation. Acetylsalicylic acid (aspirin) is an agent which has been proven to be substantially effective for this purpose.

Numerous studies of aspirin as an inhibitor of platelet aggregation have been conducted. Generally, a significant reduction of deaths in persons treated with aspirin, for myocardial infarction, has been observed. Typically, for those studies, daily dosages of aspirin in a range from about 300 milligrams to about 1,500 milligrams were used. As a result of the studies, the United States Food and Drug Administration, in its latest monograph concerning aspirin, will be reporting with favor that daily dosages of aspirin of 300 milligrams or greater lead to reduced risk of myocardial infarction. It is noted that, for the studies relied upon by the Food and Drug Administration in formulating its position, the aspirin was typically given Orally.

Apparently, aspirin, i.e. acetylsalicylic acid, irreversibly blocks the release reaction for platelet aggregation. Generally, this primarily occurs through acetylation of cyclooxygenase, by the acetylsalicylic acid, to inhibit the arachidonic acid cycle. It is generally observed that once acetylated a platelet does not aggregate. This, it is believed, is due to an inability of acetylated cyclooxygenase to initiate those chemical reactions which lead to aggregation.

Generally, for most individuals there is a complete replacement of platelets in about a 9-11 day period. That is, within a given 24 hour period approximately 10% of the platelets in a human will be replaced by new platelets.

It has been observed that for a therapeutic effect at least about 90% inhibition of normal platelet aggregation should be achieved. Generally, this has been observed to occur with dosages of aspirin in the range from 300 milligrams to 1,500 milligrams. Some studies have indicated that relatively small amounts of aspirin, for example about 40 milligrams of acetylsalicylic acid, if appropriately absorbed into the bloodstream, can substantially inhibit platelet aggregation.

When aspirin (acetylsalicylic acid) is hydrolyzed the products are salicylic acid (or a salicylate) and acetic acid (or a salt of acetic acid). This hydrolysis has been Observed to occur throughout the digestive tract with, generally, hydrolysis in the stomach and colon being substantially slower than hydrolysis in the duodenum and small bowel.

The hydrolysis can be very important to the effect of aspirin as an inhibitor of platelet aggregation. Salicylic acid, or a salicylate salt, is generally ineffective in inhibiting platelet aggregation, as it is no longer able to acetylate cyclooxygenase in the platelet. Further, there is some evidence that salicylate or salicylic acid may act to retard the ability of acetylsalicylic acid to inhibit platelet aggregration.

Both acetylsalicylic acid and salicylic acid are known to be effective analgesics. That is, whether or not it is hydrolyzed, aspirin is an effective pain killer. In the past little attention has been paid to the rate of hydrolysis taking place in different portions of the gastrointestinal tract, when aspirin is retained therein, since both the starting material and the product of the hydrolysis, once absorbed into the bloodstream, are effective as pain killers.

On the other hand, with respect to prophylactic treatment for myocardial infarction, it is particularly important that the aspirin, i.e. acetylsalicylic acid, be absorbed prior to substantial hydrolysis so that it can encounter platelets in the portal circulatory system, acetylating same and inhibiting aggregation. It is particularly important that this occur in the portal circulatory system, i.e. before the blood reaches the liver, as substantial hydrolysis will take place in the liver.

It is also desirable that an excess of acetylsalicylic acid being absorbed in the stomach and gastointestinal tract, be avoided. In particular, it is preferred that sufficiently little acetylsalicylic acid be absorbed so that any ASA which does not act upon a platelet in the portal blood system also does not survive the liver hydrolysis and make it into the systemic circulatory system. That is, excess acetylsalicylic acid beyond that which can act in the portal system to acetylate or which will be hydrolyzed by the liver, is, preferably, to be avoided. A reason for this is that acetylsalicylic acid in the systemic circulatory system, i.e. after the liver, may help encourage platelet aggregation, or at least retard the inhibitory effect of acetylsalicylic acid in the portal circulatory system.

This phenomena is generally referred to as the aspirin dilemma. That is, a dilemma is posed by the fact that the substance can both inhibit aggregation and promote aggregation (or at least inhibit inhibition), depending upon when and where it acts. The promotion of aggregation probably results from action by the aspirin in the vascular endothelium to inhibit prostacyclin formation. In general, prostacyclin, through the cyclooxygenase system, is a powerful inhibitor of platelet aggregation. Thus, prostacyclin is one of the body's natural inhibitors to platelet aggregation. Aspirin, if introduced to the systemic circulatory system still in the form of acetylsalicylic acid, may inhibit the ability of prostacyclin to have its natural effect.

It is widely recognized that aspirin can cause gastrointestinal irritation, and in fact bleeding, when given in large dosages or when giver to particularly sensitive persons. Heretofore it has not been recognized, however, that even 300 milligrams of aspirin almost universally causes some bleeding and gastrointestinal irritation when given in a form likely to le rapidly absorbed in the stomach or the upper portion of the duodenum. While in most humans this bleeding may not be sufficient to generate a great problem, statistically there will be those patients who, when treated with even only 300 milligrams of aspirin per day in a conventional manner, will undergo serious gastrointestinal bleeding and possible massive hemorrhage. This can result in serious illness or even death.

Generally the gastrointestinal irritation may be from two sources: (1) surface phenomena caused by an effect of acetylsalicylic acid on stomach mucosa; and (2) an effect not fully understood resulting from the presence of acetysalicylic acid in the systemic circulation system. With respect to the latter, it has been observed by some that aspirin, when given intravenously, can cause intestinal bleeding. This, it will be readily understood, provides an additional reason for ensuring that acetylsalicylic acid does not survive liver hydrolysis. That is, excess acetylsalicylic acid in the systemic circulatory system may aggravate the gastrointestinal bleeding problem.

It is noted that since the present Food and Drug Administration position will be that the minimal dosage level of aspirin shown to be effective for reduction of myocardial infarction is about 300 milligrams per day, it might be postulated by some that this could be given in more than one dose, for example two dosages of 150 milligrams each or three dosages of 100 milligrams each, in order to avoid or reduce risk of gastrointestinal bleeding. There are several problems with this. First, statistically it is preferable to have medication dispensed in one dose per day, so that there is less risk that a patient will forget to take his or her medication and so that a regular time of taking the medication can be more easily established. Also, much of the gastrointestinal bleeding problem probably results from surface action of the acetylsalicylic acid or salicylic acid on the mucosa of the stomach. If the stomach wall is not given sufficient time to reestablish following irritation, the next dose of aspirin is likely to irritate to an even greater extent. Thus, multiple doses of aspirin in the stomach throughout the day, are, preferably, to be avoided.

It is noted that for some patients, especially those who suffer stomach upset when taking aspirin, acetylsalicylic acid has been encased in a generally neutral coating. When the coating wears away due to the action of materials in the stomach or other portions of the gastrointestinal tract, the aspirin is released and absorbed. Generally, conventional coatings have been such as to permit aspirin absorption in the duodenum or upper part of the small bowel.

This method of dispensing aspirin is not fully acceptable for treatment of myocardial infarction, for at least several reasons:

For example, a single daily 300 milligram bolus, even when released in the duodenum, might be sufficient to substantially irritate. However, the extent of such irritation would be relatively hard to measure due to its location in the duodenum and small bowel, and therefore it would be difficult to predict or determine in which patients such irritation, if it occurs, would be likely to be hazardous. Also, any problem with irritation may be enhanced by refluxing of substantial amounts of the aspirin from the duodenum into the stomach.

Further, aspirin is generally found to hydrolyze at a fairly rapid rate in the small bowel, relative to the stomach, thus a greater amount of salicylic acid would typically be generated and absorbed into the bloodstream. This would likely inhibit the prophylactic effect of aspirin with respect to myocardial infarction in at least two manners. First, less acetylsalicylic acid would be present for acetylation of platelets in the portal circulatory system; secondly, more salicylic acid would be present in the portal circulatory system, where acetylation is to take place, and, salicylic acid, in excess, inhibits effective acetylation and thus retards effective inhibition of aggregation.

Another manner in which a single, coated, bolus of aspirin released in the gastrointestinal tract is undesirable is that throughout the day approximately 10% of the blood platelets will be replaced by the average human, with the actual percentage possibly ranging considerably from person to person depending, in part, upon the state of the person's health and variations in individual biological systems. Thus, it could be that within a 24 hour period sufficient non-acetylated platelets will have been formed to lead to a substantial risk of platelet aggregation.

It is an object of the present invention to provide a method of prophylactic treatment for myocardial infarction by the administration of acetylsalicylic acid to human patients. In particular it is an object to provide a preferred treatment whereby acetylsalicylic acid is administered to inhibit platelet aggregation, primarily through the acetylation of cyclooxygenase.

It is a further object of the present invention to achieve a preferred method of treatment, by the administration of acetylsalicylic acid wherein a a total of about 300 milligrams of acetylsalicylic acid may be administered to a patient, in a single dose or pill, given once per day.

It is still a further object of the present invention to utilize a single pill containing acetylsalicylic acid in a manner enabling at least two pulses of acetylsalicylic acid to be released into the portal circulatory system within a 24 hour period following taking of the medication. It is particularly an object to have a first pulse of aspirin be released for absorption within about two hours of the initial taking of the pill and to have a second pulse or release of aspirin occur, from the same pill, within about six to eighteen hours, and preferably about twelve hours after the initial administration of the medication.

It is a further object of the present invention to provide a method for administration of acetylsalicylic acid as a prophylactic treatment for myocardial infarction in which an initial pulse of acetylsalicylic acid is absorbed from the gastrointestinal tract in the area of the stomach or the upper portion of the duodenum and whereby a second pulse of acetylsalicylic acid is absorbed in the colon. It is a particular object that such a method be provided wherein a relatively small amount of acetylsalicylic acid is exposed to absorption in the small bowel, where substantial hydrolysis may take place.

It is still a further object of the present invention to provide such a method wherein sufficient acetylsalicylic acid is absorbed during each of the first pulse and second pulse to achieve maximum interaction of acetylsalicylic acid with platelets in the patient so that the aggregation is inhibited by 90% to 100%.

It is yet a further object of the present invention to provide such a method wherein during neither the absorption of the first pulse of acetylsalicylic acid nor the absorption of the second pulse of acetylsalicylic acid sufficient quantities of acetylsalicylic acid are likely to be absorbed into the portal circulatory system at such a rate as to provide a substantial probability of having sufficient acetylsalicylic acid likely to pass through the liver, unhydrolyzed, to be a problem either in preventing prostacyclin from inhibiting platelet aggregation, or otherwise. In particular, it is preferred that the method provide for administration of acetylsalicylic acid under conditions wherein substantially no acetylsalicylic acid will be found in the systemic circulatory system of most patients.

It is yet another object of the present invention to provide for a method of prophylactic treatment utilizing acetylsalicylic acid, for myocardial infarction, wherein gastrointestinal bleeding, especially stomach bleeding, is substantially avoided or at least its risk is substantially reduced for most patients.

It is still another object of the present invention to provide a general acetylsalicylic acid preparation characterized by the ability to release an initial pulse of aspirin in the stomach and a second pulse of aspirin in the colon, of a typical human treated with such a preparation. It is a particular object to provide such a preparation capable of releasing an initial pulse, in the stomach, of about 100 milligrams of acetylsalicylic acid and a second pulse, in the colon, of about 200 milligrams of acetylsalicylic acid. It is a further object of the present invention to provide such a new preparation wherein a pill is provided which includes an exposed outer layer of acetylsalicylic acid and which includes a coated central core of acetylsalicylic acid, wherein the exposed layer of acetylsalicylic acid is available for absorption in the stomach or the upper portion of the duodenum and the coating is such as to generally insure that the pill will make its way into the human colon prior to sufficient deterioration of the coating as to allow the second pulse of acetylsalicylic acid to be released.

It is yet another object of the present invention to provide a method of prophylactic treatment for myocardial infarction, through the administration of acetylsalicylic acid to inhibit platelet aggregation, which is particularly effective and easy to put into effect, and which can be accomplished with a single daily administration of a preparation which is particularly effective, efficient and well suited to its proposed usages.

Other objects and advantages of this invention will become apparent from the following descriptions wherein are set forth by way of illustration and example, certain embodiments and applications of this invention.

Brief Description of the Drawings

Fig. 1 is a graph showing aspirin release of a composition according to the present invention, as is described in Example 3 below.

Fig. 2 is a graph showing a modified aspirin release, as is described in Example 4 below.

Description of the Preferred And Alternative Embodiments

A method is described and reported herein for the administration of acetylsalicylic acid (ASA) to a human patient in a manner particularly suited for taking advantage of the effectiveness of acetylsalicylic acid in inhibiting platelet aggregation, while at the same time generally avoiding undesirable effects or at least substantially minimizing them. Also, a new preparation for achieving the proposed method is presented.

Two heretofore unreported observations are significant with respect to an understanding of the proposed method. The first is my observation that 300 milligrams of aspirin given once daily is sufficient to produce substantial gastrointestinal irritation, by way of bleeding, almost universally. That is, in almost any patient receiving a daily 300 milligram dose of aspirin, according to conventional methods, gastrointestinal bleeding will result. This, of course, may be complicated by the fact that the ASA is not only inducing bleeding, but it is also inhibiting clotting.

The second is my heretofore unreported observation that, for most persons, no substantial gastrointestinal bleeding is likely to be found when between about 80 and 120 milligrams of aspirin (acetylsalicylic acid) are made available in the stomach or the upper portion of the duodenum for absorption.

An initial goal of the present invention, then, is achieved by an initial provision of between about 80 and 120 milligrams of aspirin (acetylsalicylic acid) in the stomach or the upper portion of the duodenum for relatively rapid absorption. Generally it will be preferred that this initial pulse of aspirin be provided in the stomach, wherein hydrolysis of the acetylsalicylic acid, to salicylic acid, is slow relative to hydrolysis in the duodenum or small bowel.

Preferably, the initial amount of acetylsalicylic acid made available for absorption in the stomach will be about 100 milligrams. This amount will take advantage of the different dose response curves for acetylsalicylic acid with respect to: (1) gastrointestinal irritation; and, (2) effective platelet aggregation inhibition by absorbed acetylsalicylic acid. In particular, about 100 milligrams, or between 80 and 120 milligrams, of acetylsalicylic acid available in the stomach for absorption is a sufficiently small amount to be unlikely to cause substantial irritation or bleeding in most human patients. At the same time it is a sufficiently large amount to be likely to inhibit at least 90%, and generally substantially 100%, of all platelets in the body, by action on platelets in the portal circulatory system prior to passage of the acetylsalicylic acid through the liver.

Further, the initial pulse of between about 80 and 120 milligrams of aspirin is a sufficiently small amount as to be unlikely to result in any acetylsalicylic acid surviving hydrolysis in the liver. That is, it is unlikely that any acetylsalicylic acid absorbed during the initial pulse will be found in the systemic circulatory system. Even if a trace amount is found in the systemic circulatory system it will likely be in a sufficiently small amount as to be of little problem with respect to the aspirin dilemma or other complications. With respect to this it is noted that when 300 milligrams of acetylsalicylic acid are administered for absorption from a single initial pulse, some acetylsalicylic acid is detected in the systemic circulatory system. That is, some of the acetylsalicylic acid survives initial liver hydrolysis.

Herein, when it is indicated that aspirin or acetylsalicylic acid is made available in the stomach for absorption, it is meant that the acetylsalicylic acid is essentially uncoated by inhibiting agents and is present in a manner that it can be relatively rapidly absorbed and transferred into the bloodstream. It is to be understood, however, that since some hydrolysis is taking place even in the stomach the species actually absorbed will include at least both acetylsalicylic acid and salicylic acid. Generally, the amount of salicylic acid absorbed, resulting from hydrolysis in the stomach, will be relatively small and in sufficiently low quantity as to not substantially interfere with inhibition of platelet aggregation by the acetylsalicylic acid.

As part of the instant invention it is suggested that a second pulse of aspirin be made available for absorption into the portal circulatory system during a time period between about six hours and eighteen hours after the initial administration of the acetylsalicylic acid preparation. In this manner the approximate 10% replacement of platelets which will occur in the average human during a 24 hour period will not pose a substantial problem. That is, the newly absorbed acetylsalicylic acid can acetylate newly formed platelets, inhibiting same from aggregation.

In a preferred application of the present invention, the second pulse is absorbed in the colon, i.e. the large intestine. Preferably, the second pulse includes about 200 milligrams of acetylsalicylic acid, that is the difference between about 300 milligrams and the 80 to 120 milligrams absorbed in the stomach. Thus, the Food and Drug Administration's recommendation of at least 300 milligrams of acetylsalicylic acid, within a 24 hour period, is accommodated.

Preferably the second pulse of aspirin is released in the large intestine, that is the colon, for numerous reasons. First, the preferred timing, six to eighteen hours after initial ingestion of the preparation, will, for most persons, ensure that the pill has in fact moved that far down the digestive tract for absorption. Secondly, agents in the colon generally produce a lower rate of hydrolysis than do agents in the small bowel between the stomach and the colon. In part, this is due to the fact that the colon contains less of the hydrolyic enzyme. Thus, less of the acetylsalicylic acid will be hydrolyzed to salicylic acid, making more ASA available for inhibition of platelet aggregation and also resulting in a reduction of the amount of excess salicylic acid. This is preferable since, in some instances, excess salicylic acid may lead to complications or reduction of the effectiveness of the treatment.

Another reason for preferring absorption in the colon is that any bleeding irritation will be easier to monitor there, and is less likely to occur there in part due to the less acidic environment. It is generally known that acetylsalicylic acid can cause substantial bleeding in the small bowel, however the extent of that bleeding is hard to measure. On the other hand, in Europe acetylsalicylic acid has often been given rectally, i.e. in the colon, with little reporting of problems from bleeding. Further, bleeding can be readily detected in the colon, by means of readily available equipment and techniques. Thus, gastrointestinal irritation in a patient receiving a substantial dose of acetylsalicylic acid in the colon can be readily monitored and controlled.

Further, the colon for an average human is of relatively large volume and acetylsalicylic acid contained therein would be substantially diluted when absorbed. Thus, the approximately 200 milligrams of acetylsalicylic acid will be diluted somewhat prior to absorption into the portal circulatory system, delivering the acetylsalicylic acid to the liver somewhat more slowly than delivery from the stomach or small bowel. This results in a reduced chance that acetylsalicylic acid will survive liver hydrolysis and make its way into the systemic circulatory system.

Another advantage in having the second pulse released in the colon relates to gastrointestinal irritation caused by acetylsalicylic acid (ASA). Irritation of the stomach and gastrointestinal tract, by ASA, is believed, in part, to be a surface phenomenon, due in part to the acidic conditions. In time, following irritation, the surface mucosa reestablishes itself. However, if the irritated surface is presented with more irritating agent, that is the ASA, too soon increased irritation may result. Thus, with the instant method, advantage is achieved from the fact that within a 24 hour period the two pulses are released at separate points in the gastrointestinal tract. When the method is practiced, the stomach is only exposed to a relatively small amount of ASA, for absorption, once per day.

A new acetylsalicylic acid preparation proposed for use with methods according to the present invention comprises a pill having a core of acetylsalicylic acid surrounded by a protective coating, the protective coating having more acetylsalicylic acid adhering to an outer portion thereof. Preferably the outer layer of acetylsalicylic acid is made available to the stomach for absorption almost immediately upon swallowing of the pill. In the preferred embodiment this outer layer includes between about 80 and 120 milligrams of acetylsalicylic acid.

The protective coating, on the other hand, would preferably be such as to protect the inner core of acetylsalicylic acid from absorption into the portal circulatory system, at least until the pill has reached the colon and/or until between about six and eighteen hours has passed.

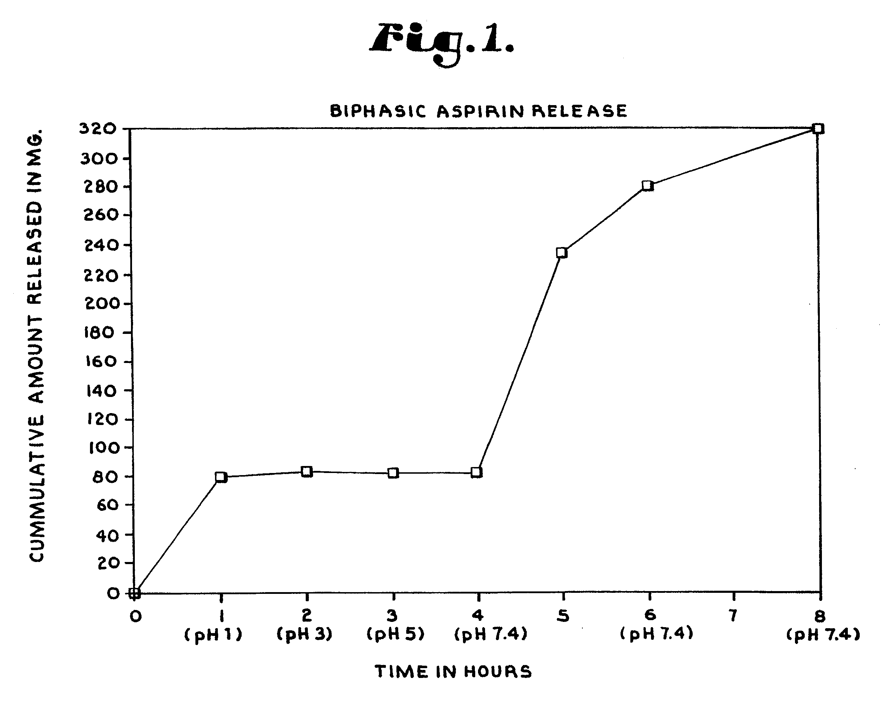

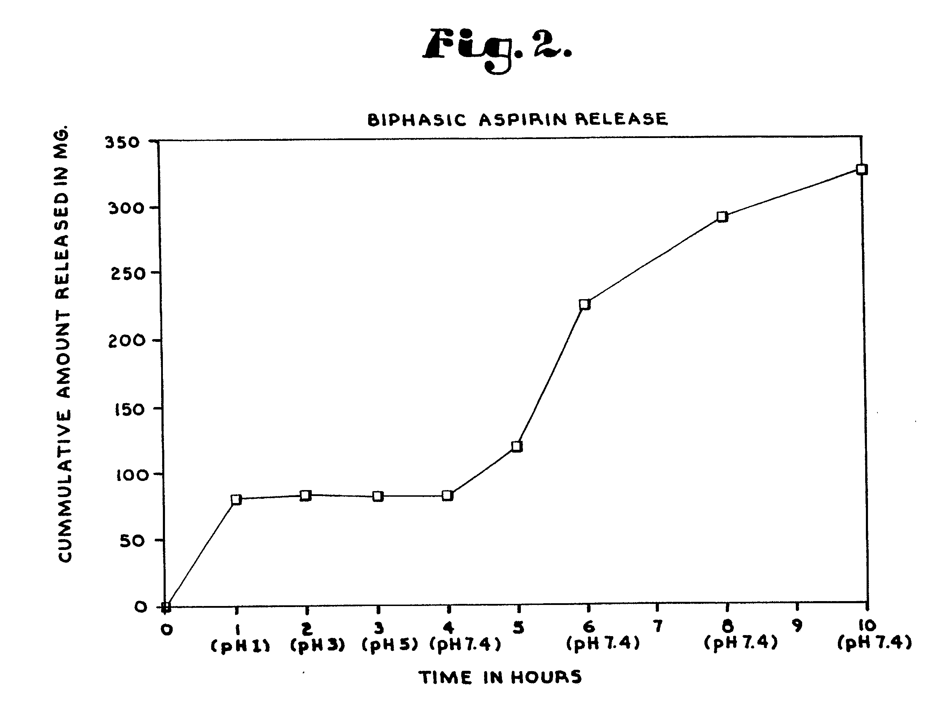

According to the present invention a variety of types of coatings for the inner core may be used. Generally, the characteristics of the coating will be such that normal agents in the gastrointestinal tract will slowly deteriorate the coating, making the internal core of acetylsalicylic acid available for absorption, as the pill moves from the stomach through the duodenum and small bowel into the colon. Alternatively, the coating will be such that it will not deteriorate in the acidic environments of the stomach, duodenum and small bowel. Upon reaching the pH neutral or slightly alkaline environment of the colon, the coating will dissolve, releasing the second pulse of acetylsalicylic acid (See Figs. 1 and 2 for example's of this process).

Preferably, the protective coating is an otherwise relatively neutral material. That is, the products from its deterioration will be essentially Waste products not likely to harm or irritate the individual. Under certain circumstances a coating which is likely to have a beneficial effect, as it deteriorates, may be used. It is foreseen that for some applications of the instant invention conventional pill coatings such as methyl cellulose, ethyl cellulose, hydroxy propyl cellulose and cellulose acetate phthalate may be used.

It is also noted that many bacteria reside in the colon that do not reside further up the gastrointestinal tract. Thus, under certain circumstances, it may be desirable to provide a coating which may be effectively deteriorated by the colon-residing bacteria, but not by other elements in the gastrointestinal tract. In this way, a coating which specifically would be unlikely to be deteriorated until the preparation passes into the colon will be obtained.

For those patients who suffer from uncomfortable symptoms of stomach upset when they take aspirin, an outer protective coating, of a conventional type, may be applied to a preparation such as that described above, delaying absorption of the initial acetylsalicylic acid pulse until passage of the preparation into the duodenum has occurred. It is noted that at this point a higher percentage of hydrolysis is likely to take place, which, as indicated above, may have undesirable effects in some instances. However, it is forseen that the regaining acetylsalicylic acid absorbed will be substantially effective in inhibiting platelet aggregation, with most of the benefits of the present invention being realized. Thus, while this variation is not preferred, it may facilitate treatment of those patients who otherwise find daily administration of acetylsalicylic acid uncomfortable.

It is foreseen that the instant invention may be applied with buffered aspirin used in either the central core, the outer layer, or both. Generally, it will be less often preferred, since it may, long term, enhance renal impairment.

The outer, immediately absorbable, layer of acetylsalicylic acid may be associated with the protected core in several manners. For example, it might completely enclose the central core. Alternatively, it might be adhered thereto, as for example on one side or to the individual aspirin crystals which are then mixed with uncoated crystals. Any of these manners, or related manners, are expected to be effective, provided the result is that the pulses are released where and when desired.

In some applications of the present invention two pills might be taken simultaneously, with the first being a pill dissolvable in the stomach or the upper portion of the duodenum to release the initial pulse into the portal circulatory system. The second pill would be a protected acetylsalicylic acid, prevented from absorption until about six to eighteen hours after the initial pulse, or until the pill makes its way into the colon.

While this latter suggested method, according to the present invention, would be expected to be effective in inhibiting myocardial infarction, while at the same achieving many of the goals above described, it would be less desirable as it would require the taking of more than one pill, with appropriate coordination in the taking of the pills, and thus would suffer in its effectiveness with those patients unwilling or unable to use the appropriate care in their daily regimen.

It is to be understood that while the method described herein has been for prophylactic treatment with respect to myocardial infarction, the method may generally be used wherever and whenever platelet aggregation is to be inhibited. That is, the method may be used for purposes other than inhibition of myocardial infarction.

Under certain circumstances it may be desirable to add additional medications to the acetylsalicylic acid preparation. These medications may be introduced either to enhance prophylactic treatment of myocardial infarction, or merely be added for treatment of other concerns or disorders. It will be understood that the additional medication may be contained either within the outer immediately absorbable layer or the inner core, or both, as desired. Generally almost any medication will be well tolerated as long as it does not interfere with the ability of acetylsalicylic acid to be absorbed or to inhibit platelet aggregation.

Generally, the instant invention has been described with respect to a 300 milligram daily dosage of. acetylsalicylic acid. A primary reason for this is to accommodate the U. S. Food and Drug Administration's position that the claim of beneficial effects from daily acetylsalicylic acid administration, with respect to prophylaxis of myocardial infarction, can be made only for dosages of between 300 milligrams and 1,500 milligrams. Thus, a goal of the present invention was to achieve the desired effects, and to minimize the undesirable consequences, while maintaining at least a 300 milligram per day dosage level. Under certain circumstances it may be, or may become, desirable to utilize the two pulse approach described herein with a dosage amount of less than 300 milligrams. In particular, it is forseen that an initial pulse of about 80 to 120 milligrams, followed by a second pulse of between about 80 and 120 milligrams will be effective.

It is also foreseen that the first pulse could be less and/or the second pulse more in certain circumstances, especially for certain uses of the drug (such as to treat migraine or atrial defibrillation), for usage with children or the like. For example, the initial pulse could be as low as 35 to 40 milligrams with the second pulse being of a like amount or much greater (such as up to 260 milligrams or more).

The following paragraphs present a detailed description of proposed treatments according to the present invention:

## EXAMPLE 1

An acetylsalicylic acid preparation is made according to the present invention. The preparation is characterized as being a pill having a central core comprising acetylsalicylic acid which can be readily dissolved by materials found in the gastrointestinal tract and which can be absorbed into the portal circulatory system. The central core comprises about 200 milligrams of acetylsalicylic acid.

The central core of acetylsalicylic acid is surrounded by a protective coating sufficient to prevent gastrointestinal juices or materials from substantially accessing the central core for a period of about twelve hours following taking of the pill by a typical human patient or until the pill has made its way into the colon. The protective coating is formed from a neutral material which can be slowly destroyed by agents located in the gastrointestinal tract, with the result being that about twelve hours will pass before substantial exposure of the acetylsalicyclic acid core for absorption. Alternatively, the protective coating may be formed from a material which can only be substantially degraded by materials in the colon, such as colon-residing bacteria.

Adjoining the protective coating is a plug of about 100 milligrams of acetylsalicylic acid. The plug may either surround the protective coating or be attached to a portion thereof. The acetylsalicylic acid outer layer is in such a state that it will be absorbed relatively rapidly in the stomach after ingestion of the pill by the patient.

The typical patient to be given a regimen according to the present invention is "middle aged", i.e. is over 45, or has a family history of severe cardiovascular disease, stroke or myocardial infarction. Statistically, the patient is more probably male or postmenopausal female.

It has been observed that the peak time period for heart attacks is at about 9 o'clock in the morning. Therefore, while the single daily dose of 300 milligrams acetylsalicylic acid may be given at almost any time of the day, a preferred method of treatment involves orally taking the medication sometime in the early evening. For the typical patient, the approximate twelve hour delay prior to the second pulse will insure that by the morning the patient has received the second pulse of acetylsalicylic acid, substantially resulting in 100% inhibition of platelet aggregation. This should lead to statistically maximal or near maximal beneficial effects.

## EXAMPLE 2

For a patient who observes substantial stomach upset from the release of substantial quantities of acetylsalicylic acid therein, a preparation is prepared according to Example 1, and the preparation is coated with a protective coating of a neutral substance sufficient to ensure that the outer layer of acetylsalicylic acid, i.e. the non-core layer, is not substantially released until the preparation has made its way into the duodenum or upper regions of the small bowel. While a higher percentage of hydrolysis is likely to take place in this location, thus inhibiting somewhat the effectiveness of the treatment, the patient will still receive substantial beneficial effect from aspirin inhibition of platelet aggregation.

## EXAMPLE 3

An acetylsalicylic acid preparation was made according to the present invention. It was prepared as follows: 3500 grams (g.) of aspirin crystals were charged into a 12 inch Wurster type fluid bed coater (Glatt GPGC-5). The aspirin crystals were fluidized and coated with an anionic acrylic polymer solution prepared by dissolving 1500 g. of methaerylic acid copolymer USP/NF (Eudragit S-100 of Rhom Parma. Co.) in 1200 milliliters (ml.) of methylene chloride and 300 ml. of methanol. 211 g. of the coated aspirin wae then mixed with 114 g. of uncoated aspirin. The mix was encapsulated in hard gelatin capsules to a fill weight of 430 milligrams (mg.) so as to result in 325 mg. of total aspirin per capsule.

The biphasic profile of dissolution of the capsule when tested in a dissolution medium with gradual pH changes (i.e. pH 1.0 at hour 1, pH 3.0 at hour 2, pH 5.0 at hour 3, and pH 7.4 at hour 4 and beyond) is shown in Fig. 1.


EXAMPLE 4


An acetylsalicylic acid preparation was made according to the present invention. It was prepared as follows: 3500 g. of aspirin crystals were charged into a 12 inch Wurster type fluid bed coater (Glatt GPGC-5). The aspirin crystals were fluidized and coated with an anionic acrylic polymer solution prepared by dissolving 1500 g.of methaerylic acid copolymer USP/NF (Eudragit S 100 of Rhom Pharma. Co.) in 1200 ml. of methylene chloride and 300 ml. of methanol. The coated crystals were further coated with 25 g. of ethylcellulose dissolved in 300 ml. of methylene chloride. 210 g. of the coated crystals were then mixed with 115 g. of uncoated aspirin. This mixture was encapsulated in hard geletin capsules to a fill weight of 432 mg. to result in 325 mg. of aspirin per capsule.

When tested in a dissolution medium with gradual pH changes (as in EXAMPLE 3), the final mixture in capsule provided a biphasic drug release profile shown in Figure 2.

It is to be understood that while certain embodiments and examples of the present invention have been illustrated or described herein, it is not to be limited to the specific forms herein described and shown, except as limited by the Claims.


**Claims**

1. A preparation for administration to effect substantial reduction of blood platelet aggregation in human patients; said preparation comprising:
   (a) a central core of acetylsalicylic acid;
   (b) a protective coating surrounding said central core of acetylsalicylic acid designed to deteriorate as said coating travels through the gastrointestinal tract, thereby releasing said central core in the colon; and
   (c) an outer layer of acetylsalicylic acid attached to said protective coating.

2. The preparation according to claim 1, wherein: said protective coating is sufficient to prevent absorption of said central core of acetylsalicylic acid until a time period of between about six to eighteen hours after the administration of the preparation.

3. The preparation according to claim 1 or 2, wherein: said protective coating prevents substantial absorption of the central core of acetylsalicylic acid until said central core of acetylsalicylic acid is in the colon.

4. The preparation according to any one of claims 1, 2 and 3, wherein:
   (a) said preparation contains a total of about 300 milligrams of acetylsalicylic acid.

5. A preparation according to claim 4, wherein:
   (a) said central core contains between about 180 and 220 milligrams of acetylsalicylic acid; and
   (b) said outer layer contains between about 80 and 120 milligrams of acetylsalicylic acid.

6. A preparation according to claim 5, wherein: said central core contains about 200 milligrams of acetylsalicylic acid; and said outer layer contains about 100 milligrams of acetylsalicylic acid.

7. A preparation according to any one of the preceding claims, wherein: said preparation contains an outer stomach-protective coating preventing absorption of the outer layer of acetylsalicylic acid until the pill has made its way through the stomach into at least the duodenum.

8. A preparation according to any one of the preceding claims, wherein: said outer layer of acetylsalicylic acid completely surrounds said central core and protective coating.

9. A preparation according to any one of claims 1 to 7, wherein: said outer layer of acetylsalicylic acid is adhered to, but does not completely surround, said central core and said protective coating.

10. A preparation according to any one of the preceding claims, wherein:
   (a) said protective coating is composed of a material subject to degradation by colon-residing bacteria but substantially resistant to degradation by the chemical environment of the stomach and small bowel.

11. A preparation for administration to effect substantial reduction of blood platelet aggregation in a human patient; said preparation comprising:

(a) acetylsalicylic acid crystals; and

(b) a protective coating surrounding said crystals; said coating being pH sensitive and will substantially remaining intact in a stomach, a duodenum and a small bowel of the patient and said coating dissolving upon reaching the colon; combined with

(c) uncoated acetylsalicylic acid crystals in a mixture.

12. The preparation according to claim 11, wherein: said preparation contains a total of about 300 milligrams of acetylsalicylic acid.

13. A preparation according to claim 12, wherein:

(a) said crystals with said protective coating total about 180 and 220 milligrams of acetylsalicylic acid; preferably about 200 milligrams of acetylsalicylic acid and

(b) said uncoated crystals total between about 80 and 120 milligrams of acetylsalicylic acid, preferably about 100 milligrams of acetylsalicylic acid.

14. A preparation according to any one of claims 11 to 13, wherein:

said preparation contains ar outer stomach-protective coating preventing absorption of the outer layer of acetylsalicylic acid until the pill has made its way through the stomach into at least the duodenum.

Fig.1.

BIPHASIC ASPIRIN RELEASE

**Fig. 2.**

BIPHASIC ASPIRIN RELEASE

EP 0 411 590 A2